# EUROPEAN PATENT APPLICATION

(11) **EP 4 772 540 A1**
(43) Date of publication of application: **08.07.2026**
(21) Application number: 24855157.4
(22) Date of filing: 22.08.2024
(51) Int. Cl.: C07K 19/00, C07K 14/025, C07K 14/035, A61K 39/295, C12N 15/62, C12N 15/63, A61P 31/20, A61P 35/00, C12R 1/93

(54) **HYBRID PROTEIN, HYBRID NUCLEOTIDE SEQUENCE, NUCLEIC ACID MOLECULE, VECTOR FOR HYBRID PROTEIN EXPRESSION, USE OF HYBRID PROTEIN OR NUCLEOTIDE SEQUENCE, FORMULATION, PROCESS FOR PRODUCING A FORMULATION AND METHOD FOR TREATING LESIONS AND/OR CANCER CAUSED BY HUMAN PAPILLOMAVIRUS (HPV) INFECTION**

(30) Priority: 23.08.2023 BR 102023016990
(71) Applicant: ImunoTera Soluções Terapêuticas Ltda, 05508000 São Paulo (BR)
(72) Inventor: RIBEIRO, Bruna Felicio Milazzotto Maldonado Porchia, 05713-470 São Paulo (BR); APS, Luana Raposo De Melo Moraes, 07781-775 Cajamar (BR)
(74) Representative: Currado, Luisa
(86) International application number: PCT/BR2024/050376
(87) International publication number: WO 2025/039059

(57) **Abstract**

The present invention describes an approach to improve immunogenic and therapeutic performance in the prevention and/or treatment of lesions and/or cancer caused by or associated with human papillomavirus (HPV). More specifically, the approach involves a hybrid protein selected from two entities with enhanced immunogenic and therapeutic potential, its use, and a formulation comprising the same. Furthermore, nucleotide sequences, vectors for the expression of recombinant proteins, are revealed, as well as a process for producing a formulation comprising said entity and a therapeutic method. The invention falls within the fields of medicine, pharmacy, and biotechnology.

## Description

### Field of the Invention

The present invention describes an approach to improve immunogenic and therapeutic performance in the prevention and/or treatment of lesions and/or cancer caused by or associated with human papillomavirus (HPV). More specifically, the approach involves a hybrid protein selected from two entities with enhanced immunogenic and therapeutic potential, its use, and a formulation comprising the same. Furthermore, nucleotide sequences, a vector for the expression of recombinant proteins, are revealed, as well as a process for producing a formulation comprising said entity and a therapeutic method. The invention falls within the fields of medicine, pharmacy, and biotechnology.

### Background of the Invention

Human papillomavirus (HPV) is primarily transmitted through sexual contact and is the most common viral infection of the reproductive tract. Most sexually active women and men will be infected at some point in their lives, and some people may experience persistent infections. When left untreated, these infections can progress to precancerous lesions and cancer in the anogenital region. HPV is considered the causative agent of cervical cancer, accounting for 95% of cases. A study published by BMC Public Health indicates that for every new case of HPV-induced cervical cancer, there are six cases of precursor lesions¹. In addition to cervical cancer, HPV is responsible for 90% of anal cancer cases, 75% of vaginal cancer cases, 70% of vulvar cancer cases, 70% of oropharyngeal cancer cases, and approximately 60% of penile cancer cases². In 2020, there were 852,620 cases of cancer caused by HPV, in both men and women. The study *"Changes of new cases from 2020 to 2040"* produced by *Global Cancer Observatory* indicates a growing trend in the number of cases of these types of cancer, which could reach 1,205,162 by 2040.

To combat this public health problem, several prophylactic and therapeutic methods are available. For example, prophylactic HPV vaccines based on VLPs (virus-like particles), such as Gardasil and Cervarix, have been developed. However, these approaches do not protect individuals in whom the infection is established or who already have precursor lesions. In these cases, the available treatments are often invasive and expensive, as well as not very specific, such as surgery, radiotherapy, and some chemotherapies. Furthermore, cancer recurrence and the development of resistance to conventional treatments are problems faced by many patients. Therefore, there is a need for new anti-tumor strategies that are safe, effective, and less traumatic for the patient.

Over the past two decades, therapeutic vaccines capable of teaching the immune system to recognize and eliminate cancerous cells in a specific way have been developed^{3,4}. In the case of precursor lesions and cancers caused by HPV-16, these vaccines have been primarily based on the expression of a chimeric protein, resulting from the genetic fusion of glycoprotein D (gD) of Herpes simplex virus type 1 (HSV-1) with the oncoprotein E7 of HPV-16^{5,4}. Furthermore, this research demonstrates that this technology can be applied in the form of DNA-based vaccines and the purified recombinant protein itself^{3,4}.

In the construction of these chimeric proteins, the gD component acts as an adjuvant, since by interacting with the HVEM cellular receptor, it promotes the activation of NF-κB and blocks co-inhibitory signals mediated by the BTLA and CD160 ligands, resulting in the activation of antigen-specific T lymphocytes⁶⁻¹⁰. Furthermore, gD can act as a molecular vector that directs antigens fused to it to populations of dendritic cells specialized in antigen cross-presentation, which are important in generating antitumor responses¹¹. Finally, contact with the gD protein delays the expression of PD-L1 in dendritic cells, allowing the immunostimulatory function of these cells to persist for a longer period.

However, this technology also has technical limitations, such as poor control of tumor growth and unsatisfactory immunogenic activity. Therefore, this technology must continue to evolve. In this sense, changes in the formulations of both of the aforementioned therapeutic vaccines, such as the use of other adjuvants^{11,13,16}, association with delivery methods¹⁴, to other agents^{12,15} and chemotherapy drugs¹⁷ are some of the improvements already described. The references below detail the state of the art mentioned.
1. Tao, L. et al. Prevalence and risk factors for cervical neoplasia: A cervical cancer screening program in Beijing. BMC Public Health 14, 1-9 (2014).
2. Dong, Z. et al. Immunodiagnosis and Immunotherapeutics Based on Human Papillomavirus for HPV-Induced Cancers. Frontiers in Immunology vol. 11 Preprint at https://doi.org/10.3389/fimmu.2020.586796 (2021).
3. Diniz, M. O., Lasaro, M. O., Ertl, H. C. & Ferreira, L. C. S. Immune responses and therapeutic antitumor effects of an experimental DNA vaccine encoding human papillomavirus type 16 oncoproteins genetically fused to herpesvirus glycoprotein D. Clin Vaccine Immunol 17, 1576-83 (2010)
4. Porchia, B. F. M. M. et al. Purified herpes simplex type 1 glycoprotein D (gD) genetically fused with the type 16 human papillomavirus E7 oncoprotein enhances antigen-specific CD8 + T cell responses and confers protective antitumor immunity. Mol Pharm 8, 2320-2330 (2011).
5. Lasaro, M. O., Diniz, M. O., Reyes-Sandoval, A., Ertl, H. C. & Ferreira, L. C. S. Anti-tumor DNA vaccines based on the expression of human papillomavirus-16 E6/E7 oncoproteins genetically fused with the glycoprotein D from herpes simplex virus-1. Microbes Infect. 7, 1541-1550 (2005).
6. Lasaro, M. O. et al. Targeting of antigen to the herpesvirus entry mediator augments primary adaptive immune responses. Nat Med 14, 205-212 (2008).
7. Sciortino, M. T. et al. Involvement of gD/HVEM interaction in NF-kB-dependent inhibition of apoptosis by HSV-1 gD. Biochem. Pharmacol. 76, 1522-1532 (2008).
8. Sciortino, M. T. et al. Involvement of HVEM receptor in activation of nuclear factor kappaB by herpes simplex virus 1 glycoprotein D. Cell. Microbiol. 10, 2297-311 (2008).
9. Steinberg, M., Cheung, T. C. & Ware, C. F. The Signaling Networks of the Herpesvirus Entry Mediator (TNFRSR14) in Immune Regulation. Immunol Rev 244, 169-187 (2012).
10. Cheung, T. C. et al. Unconventional ligand activation of herpesvirus entry mediator signals cell survival. Proc. Natl. Acad. Sci. U. S. A. 106, 6244-9 (2009).
11. Porchia, B. F. M. M. et al. Herpes simplex virus glycoprotein D targets a specific dendritic cell subset and improves the performance of vaccines to human papillomavirus-associated tumors. Mol. Cancer Ther. 16, 1922-1933 (2017).
12. Diniz, M. O., Cariri, F. A. M. O., Aps, L. R. M. M. & Ferreira, L. C. S. Enhanced therapeutic effects conferred by an experimental DNA vaccine targeting human papillomavirus-induced tumors. Hum. Gene Ther. 24, 861-70 (2013).
13. Aps, L. R. M. M. et al. Bacillus subtilis spores as adjuvants for DNA vaccines. Vaccine 33, 2328-34 (2015).
14. Sales, N. S. et al. In vivo electroporation enhances vaccine-mediated therapeutic control of human papilloma virus, associated tumors by the activation of multifunctional and effector memory CD8+ T cells. Vaccine 35, 7240-7249 (2017).
15. Moreno, A. C. R. et al. The Combined Use of Melatonin and an Indoleamine 2,3-Dioxygenase-1 Inhibitor Enhances Vaccine-induced Protective Cellular Immunity to HPV16-Associated Tumors. Front. Immunol. 9, 1-14 (2018)
16. Pagni, R. L., Silva, O., Rodrigues, K. B., Sales, S. & Moreno, C. R. 2,3-dioxygenase as potential adjuvant targets for tumors immunotherapy. Front. Immunol. 1-15 (2022).
17. Porchia, B. F. M. M. et al. Active immunization combined with cisplatin confers enhanced therapeutic protection and prevents relapses of HPV-induced tumors at different anatomical sites. Int. J. Biol. Sci. 18, 15-29 (2022).

In addition to the articles referenced above, the following documents related to the topic were found in the search for the state of the art in patent literature:

Brazilian patent PI1003749, owned by USP, protects vectors/plasmids that harbor the DNA sequence for the expression of the hybrid protein gDE7, which consists of a polypeptide segment of the HPV-16 oncoprotein E7 between two segments corresponding to a modified form of the glycoprotein D (gD) of herpes simplex type-1 (HSV-1). Furthermore, the aforementioned DNA and protein sequence is protected, as well as the use and pharmaceutical and/or veterinary formulations thereof for the treatment of infectious and/or degenerative diseases, or for the preparation of vaccine adjuvants for other antigens. It is worth noting that the protein protected by the patent is different from the proteins presented in this patent application.

Brazilian patent BR1120120049283, owned by USP, protects an isolated nucleic acid sequence and an expression vector featuring codons optimized for the expression of the HPV E7 protein fused to the HSV gD protein in mammalian cells. The document also discloses a synergistic immunogenic composition comprising the aforementioned expression vector combined with another vector encoding a cytokine for the production of vaccines for the prevention of HPV-induced tumors. Therefore, this patent differs from the present invention by using the native form of HSV-1 glycoprotein D and requiring a combination of vectors to achieve the desired effect.

Thus, based on the literature reviewed, no documents were found that anticipated or suggested the teachings of the present invention, so the solution proposed here, in the inventors' view, possesses novelty and inventive activity compared to the state of the art.

Therefore, improvements are still needed in the performance of therapeutic vaccines against lesions and/or cancer caused by HPV infection.

### Summary of the Invention

The present invention solves the problems of the prior art by providing an approach to improve immunogenic and therapeutic performance in the prevention and/or treatment of lesions and/or cancer caused by or associated with human papillomavirus (HPV).

In the present invention, a modified protein, selected from two hybrid proteins, or their corresponding nucleotide sequences, exhibits improved immunogenic and therapeutic potential. The constructions in this patent application present an absence of the signal peptide, deletion of the C-terminal portion of the gD protein comprising the transmembrane region, and the addition of extension sequences to the C- and/or N-terminal region. Furthermore, the hybrid proteins described here are administered as exogenous antigens, so that they are processed and presented in the context of MHC class I and II molecules, generating responses from CD4 and CD8 T lymphocytes, as well as antibodies. Formulations containing these proteins are stable and can be stored at refrigerated temperatures only, making distribution and storage relatively easy.

In a first object, the present invention discloses a hybrid protein comprising an extender portion linked to the N- and/or C-terminal region of a modified portion of HSV-1 glycoprotein D fused to HPV oncoprotein E7. Preferably, HPV refers to viral subtypes with oncogenic potential, more preferably HPV-16.

In a second object, the present invention presents a hybrid nucleotide sequence comprising a coding sequence of an extender moiety linked to a modified HSV-1 glycoprotein D coding sequence fused to an HPV E7 protein coding sequence.

In a third object, the present invention presents a nucleic acid molecule characterized by the nucleotide sequence of SEQ ID NO: 5, or degenerate sequences thereof, which encode the amino acid sequence of SEQ ID NO: 2.

In a fourth object, the present invention presents a nucleic acid molecule characterized by the nucleotide sequence of SEQ ID NO: 6, or degenerate sequences thereof, which encode the amino acid sequence of SEQ ID NO: 3.

In a fifth object, the present invention presents a vector for the expression of a hybrid protein, as defined above, comprising a genetic selection marker and one or more promoters functionally linked to a recombinant nucleotide sequence selected from the group consisting of SEQ ID NO: 5 or SEQ ID NO: 6.

In a sixth object, the present invention presents the use of the hybrid protein, as defined above, or the hybrid nucleotide sequence, as defined above, for the preparation of a medicament for the treatment of lesions and/or cancer caused by human papillomavirus (HPV) infection.

In a seventh object, the present invention presents a formulation comprising at least one hybrid nucleotide sequence selected from the group consisting of SEQ ID NO: 5, SEQ ID NO: 6, SEQ ID NO: 17 and/or SEQ ID NO: 18, and at least one pharmaceutically acceptable excipient.

In an eighth object, the present invention presents a formulation comprising at least one hybrid protein selected from the group consisting of SEQ ID NO: 2 and/or SEQ ID NO: 3, and at least one pharmaceutically acceptable excipient.

In a ninth object, the present invention presents a process for producing a formulation, comprising a mixing step of the hybrid protein or nucleotide sequence, as defined above, with at least one pharmaceutically acceptable excipient.

In a tenth object, the present invention presents a method for treating lesions and/or cancer caused by human papillomavirus (HPV) infection comprising administering a hybrid protein or a nucleotide sequence, as defined above.

In an eleventh object, the present invention presents a hybrid protein or a hybrid sequence, as defined above, for use as a medicament.

In a twelfth object, the present invention presents a hybrid protein or a hybrid sequence, as defined above, for use in the treatment of lesions and/or cancer caused by human papillomavirus (HPV) infection.

Degenerate sequences of the nucleotide sequences described above, which encode the same product, are also objects of the invention and should be considered within the scope of the appended claims.

These and other objects of the invention will be immediately appreciated by those skilled in the art and will be described in detail below.

### Brief Description of the Figures

The following figures are presented:

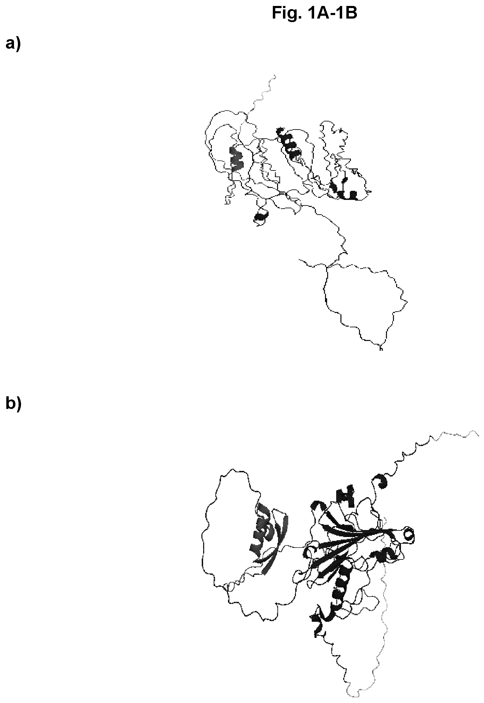

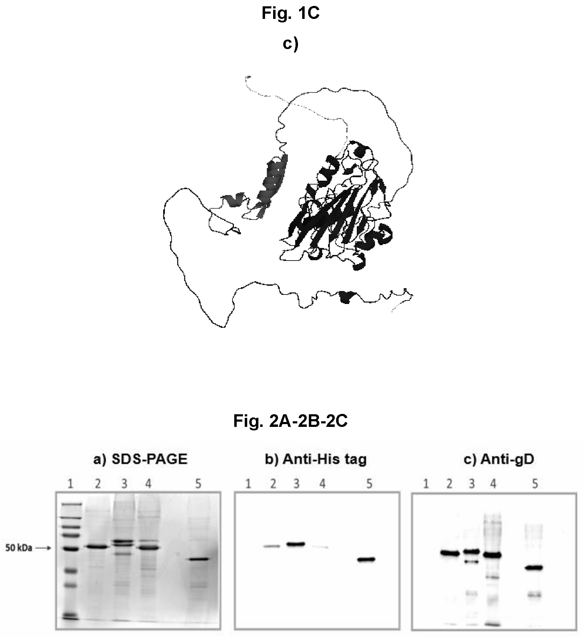

Fig. 1A, 1B and 1C show the in silico prediction of the secondary structure of the hybrid proteins with SEQ ID No: 1, SEQ ID No: 2 and SEQ ID No: 3, using the AlphaFold Protein Structure Database. Dark gray represents the gD protein; medium gray, the HPV-16 E7 oncoprotein; and light gray, the extender amino acids.
Fig. 2A, 2B and 2C show the analysis of the electrophoretic profile (Fig. 2A) and recognition of recombinant proteins by Western Blot (Fig. 2B and Fig. 2C). 1-Molecular weight marker; 2 - recombinant protein of SEQ ID No: 3; 3 - recombinant protein of SEQ ID No: 2; 4 - recombinant protein of SEQ ID No: 1; 5 - gD. Run performed on SDS-PAGE at 15% and recognition by anti-His (Thermo) and anti-gD (1D3) antibodies.

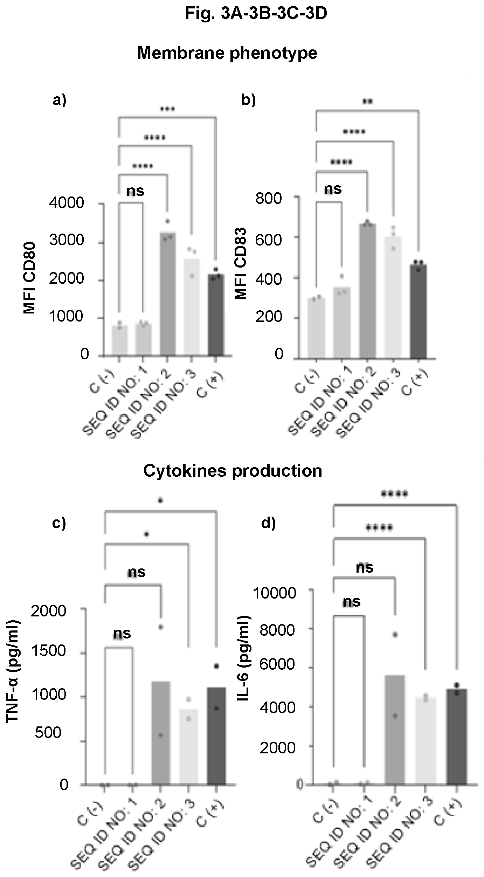

Fig. 3A, 3B, 3C and 3D show the activation capacity of monocyte-derived dendritic cells (MoDCs) mediated by different recombinant proteins, analyzed by the increased expression of surface molecules (Fig. 3A and 3B) and cytokine production (Fig. 3C and 3D). The MoDCs were incubated with the different stimuli for 48 hours. Then, membrane phenotype analysis was performed by flow cytometry and cytokine dosage by CBA (Cytometer Bead Array). The data demonstrates the average of replicates from a representative experiment out of a total of three experiments performed with similar results. One-way ANOVA and Bonferroni post-hoc test. **** p<0.0001; ** p<0.01; * p<0.05; ns = not significant.

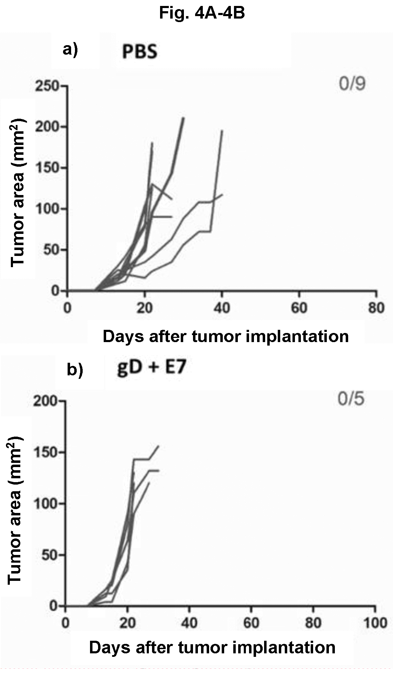

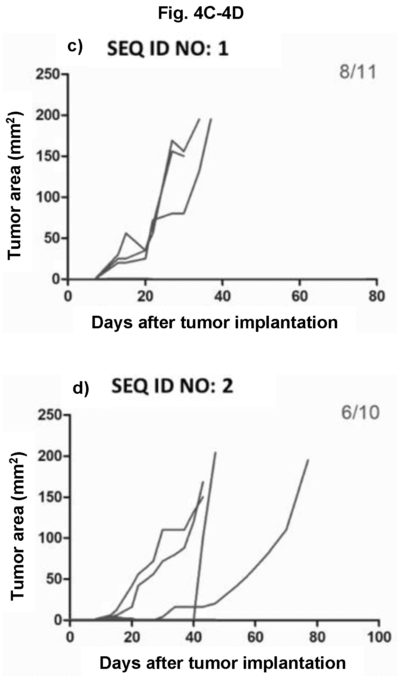

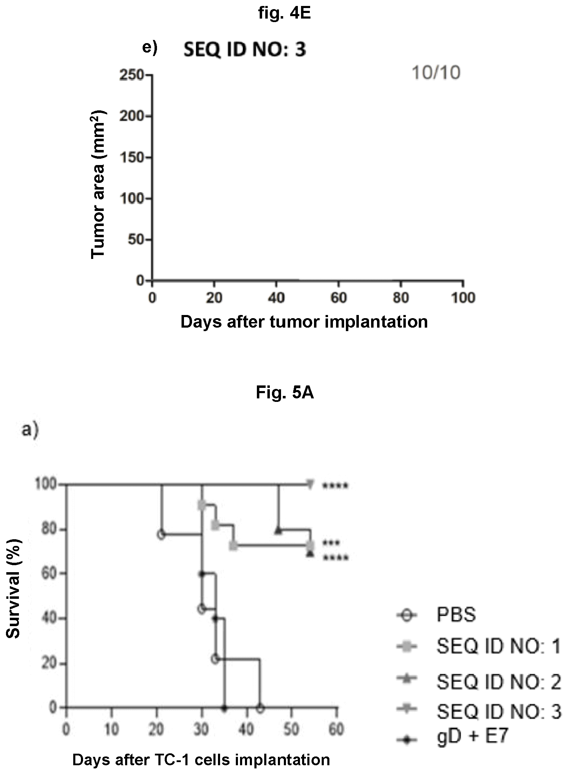

Fig. 4A, 4B, 4C, 4D and 4E show the antitumor activity of the different recombinant proteins analyzed using TC-1 cells implantation, which is the most relevant HPV-associated tumor model in C57BL/6 mice. One day after tumor implantation, the animals began treatment with two 60µg doses of each protein via subcutaneous injection, with a 7-day interval between doses. The presence of tumors was monitored for at least 60 days, with a frequency of twice a week. The major (D) and minor (d) diameters of the tumors were obtained using a caliper, and the animals were euthanized when one of the diameters reached 15 mm. The graphs show the results for tumor area, represented by D x d in mm², from two experiments performed. The number of tumor-free mice/total number of mice is represented in the upper right corner of each graph.

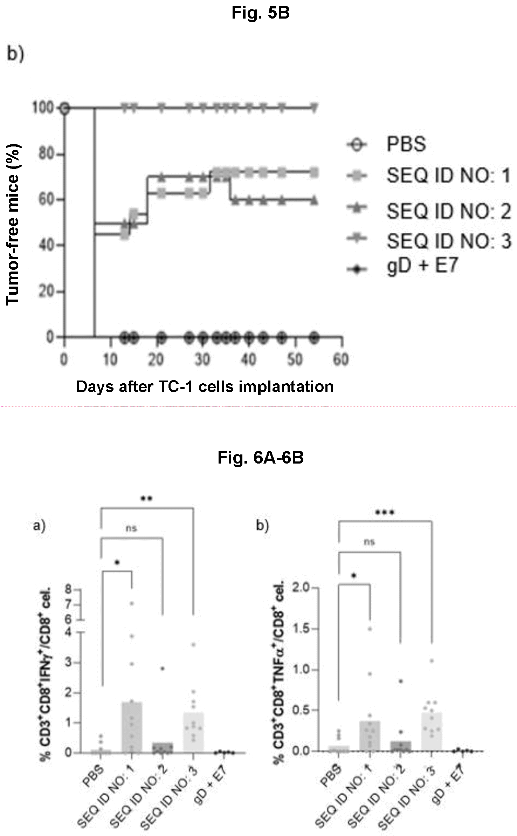

Fig. 5A and 5B show the antitumor activity of different recombinant proteins analyzed by implanting TC-1 cells (10⁵ cells), the most relevant HPV-associated tumor model in C57BL/6 mice. One day after the tumor cell implantation, the animals began treatment with two doses of 60µg of each protein via subcutaneous injection, with a 7-day interval between doses. The presence of tumors was monitored for at least 60 days, with a frequency of twice a week. The diameters of the tumors were obtained using a caliper, and the animals were euthanized when one of the diameters reached 15 mm. The graphs show the survival rate (Fig. 5A) and the frequency of tumor-free mice (Fig. 5B), resulting from two compiled experiments. Survival curve analysis: Log-rank (Mantel-Cox). **** p< 0.0001; *** p<0.001 versus PBS group.
Fig. 6A and 6B show the cellular immune response assessed using the ICS assay of peripheral blood cells from mice 7 days after the 2^{nd} dose of treatment. Approximately 1x10⁶ cells were stimulated in vitro with E7₄₉₋₅₇ peptide specific to CD8 T cells restricted to the MHC I complex (SEQ ID NO: 7) for 12 hours in the presence of CD28 and Brefeldin, labeled with anti-CD3, -CD8, IFN-γ or TNFα antibodies and evaluated by flow cytometry. The frequency of CD3⁺CD8⁺IFN-γ⁺ cells (Fig. 6A) and CD3⁺CD8⁺TNFα⁺ cells (Fig. 6B) represents the proportion of CD8 T lymphocytes that respond specifically against tumor cells during the evaluated period. One-way ANOVA and Bonferroni post-test. *** p<0.001; ** p<0.01; * p<0.05; ns = not significant.

### Detailed Description of the Invention

The present invention provides an approach to improve immunogenic and therapeutic performance in the prevention and/or treatment of lesions and/or cancer caused by or associated with human papillomavirus (HPV).

The hybrid proteins presented herein comprise modifications in the sequence of the HPV-16 E7 oncoprotein and/or the HSV-1 glycoprotein D. These modifications include: a) point mutations (C24G and E26G) in the E7 protein to prevent its interaction with its cellular targets, thus avoiding the oncogenic potential of E7; b) exclusion of the C-terminal portion of glycoprotein D to improve the production, purification, and affinity of the molecule to the HVEM cellular receptor; c) addition of histidine tails to the N- and/or C-terminal portion; and d) addition of extender amino acids to the N- and/or C-terminal portion to stabilize the sequence and promote interaction of the protein with the HVEM cellular receptor. Thus, the invention provides control over tumor growth and increased activation capacity of human dendritic cells differentiated from monocytes.

Furthermore, the nucleotide sequence defined by SEQ ID NO: 6 features codons optimized for expression in *E. coli,* increasing production yield by up to 15 times when compared to the expression of the hybrid protein encoded by SEQ ID NO: 5.

Advantageously, the protein formulations according to the present invention are easily evaluated in vitro for biological activity in various contexts. For example, in immune cells of patients with different diseases caused by HPV (precursor lesions or cervical cancer, anal cancer, oropharyngeal cancer, among others).

The demonstrated biological activity shown in the examples of the present invention is evidence that the immune system activation mechanism mediated by these proteins is maintained and indicates therapeutic efficacy in different clinical contexts. This characteristic contributes to the personalized treatment of patients.

In a first object, the present invention presents a hybrid protein comprising an extender portion linked to the N- and/or C-terminal region of a modified portion of HSV-1 glycoprotein D fused to HPV oncoprotein E7. Preferably, HPV refers to viral subtypes with oncogenic potential, more preferably HPV-16.

In one embodiment, this hybrid protein comprises a sequence AA₁-AA₂-(AA₃)ₙ-(AA₄)ₙ-AA₅-(AA₃)ₙ-(AA₄)ₙ-(AA₆)ₘ-(AA₇)ₚ-AA₈-(AA₉)_{q}-(AA₁₀)ᵣ-(AA₁₁)ₛ, wherein:
AA₁ is an extender portion containing between 10 and 35 amino acids;
AA₂ is a portion of HSV-1 glycoprotein D between amino acids 26 to 269 or 21 to 269;
AA₃ is a nonpolar amino acid;
AA₄ é a polar amino acid;
AA₅ is a portion of HPV E7 antigen between amino acids 1 to 98, in which, optionally, the residues at positions 24 and 26 are replaced by a nonpolar amino acid;
AA₆ is a nonpolar amino acid;
AA₇ is a polar amino acid;
AA₈ is a portion of HSV-1 glycoprotein D between amino acids 270 to 341 or 268 to 339;
AA₉ is a nonpolar amino acid;
AA₁₀ is an acidic amino acid;
AA₁₁ is a tail containing 6 histidines
n, m, p, q, r, s are, independently, an integer from 0 to 1.

The nonpolar amino acids consist of alanine, glycine, isoleucine, leucine, phenylalanine, proline, tryptophan, valine, and methionine.

The polar amino acids consist of asparagine, glutamine, cysteine, tyrosine, threonine, and serine.

The acidic amino acids consist of aspartic acid or glutamic acid.

In one embodiment, the extension portion is defined by SEQ ID NO: 8 or SEQ ID NO: 13.

In one embodiment, the hybrid protein is defined by SEQ ID NO: 2 or SEQ ID NO: 3.

Specifically, the hybrid protein with SEQ ID NO: 2 is formed by: i) an extension amino acid sequence and a histidine tail (SEQ ID NO: 8), followed by ii) a first segment of HSV-1 glycoprotein D corresponding to amino acids 21 to 269 (SEQ ID NO: 9) linked to the sequence of iii) HPV-16 E7 antigen corresponding to amino acids 1 to 98 of the viral protein (SEQ ID NO: 10), which in turn is linked to iv) the second segment of HSV-1 glycoprotein D corresponding to amino acids 268 to 339 (SEQ ID NO: 11) followed by a v) second sequence of extender amino acids comprising a histidine tail (SEQ ID NO: 12).

Specifically, the hybrid protein with SEQ ID NO: 3 is formed by: i) an extender amino acid sequence and a histidine tail (SEQ ID NO: 13), followed by ii) a first segment of HSV-1 glycoprotein D corresponding to amino acids 26 to 269 (SEQ ID NO: 14) linked to the sequence of iii) HPV-16 E7 antigen corresponding to amino acids 1 to 98 of the viral protein, wherein cysteine and glutamic acid at positions 24 and 26, respectively, have been replaced by glycine (SEQ ID NO: 15); In turn, this sequence is linked to the iv) second segment of HSV-1 glycoprotein D corresponding to amino acids 270 to 341 (SEQ ID NO: 16). Between portions ii)-iii) and iii)-iv) are the amino acids LQ and LQGT, respectively.

In a second object, the present invention presents a hybrid nucleotide sequence comprising a coding sequence of an extender moiety linked to a coding sequence of a modified HSV-1 glycoprotein D fused to a coding sequence of the HPV E7 protein.

In one embodiment, the hybrid nucleotide sequence is defined by SEQ ID NO: 19, wherein:
R is A or G;
Y is C or T;
H is A, C or T;
N is A, C, G or T;
T is thymine; and

Optionally, one or more AGY codons are replaced by TCN, one or more TTR are replaced by CTN, and one or more CGN are replaced by AGR.

In one embodiment, this hybrid nucleotide sequence is a DNA nucleotide sequence defined by SEQ ID NO: 5.

In one embodiment, the hybrid nucleotide sequence is defined by SEQ ID NO: 23, wherein:
R is A or G;
Y is C or T;
H is A, C or T;
N is A, C, G or T;
T is uracil; and

Optionally, one or more AGY codons are replaced by TCN, one or more TTR are replaced by CTN, and one or more CGN are replaced by AGR.

In one embodiment, this hybrid nucleotide sequence is an RNA nucleotide sequence defined by SEQ ID NO: 17.

In another embodiment, the hybrid nucleotide sequence is defined by SEQ ID NO: 20, wherein:
R is A or G;
Y is C or T;
H is A, C or T;
N is A, C, G or T;
T is thymine; and

Optionally, one or more AGY codons are replaced by TCN, one or more TTR are replaced by CTN, and one or more CGN are replaced by AGR.

In one embodiment, this hybrid nucleotide sequence is a DNA nucleotide sequence defined by SEQ ID NO: 6.

In another embodiment, the hybrid nucleotide sequence is defined by SEQ ID NO: 24, wherein:
R is A or G;
Y is C or T;
H is A, C or T;
N is A, C, G or T;
T is uracil; and

Optionally, one or more AGY codons are replaced by TCN, one or more TTR are replaced by CTN, and one or more CGN are replaced by AGR.

In one embodiment, this hybrid nucleotide sequence is an RNA nucleotide sequence defined by SEQ ID NO: 18.

In a third object, the present invention presents a nucleic acid molecule characterized by the nucleotide sequence of SEQ ID NO: 5, and degenerate sequences thereof, which encode the amino acid sequence of SEQ ID NO: 2.

In a fourth object, the present invention presents a nucleic acid molecule characterized by the nucleotide sequence of SEQ ID NO: 6, and degenerate sequences thereof, which encode the amino acid sequence of SEQ ID NO: 3.

In a fifth object, the present invention provides a vector for the expression of a hybrid protein, as defined above, comprising a genetic selection marker and one or more promoters functionally linked to a recombinant nucleotide sequence selected from the group consisting of SEQ ID NO: 5 or SEQ ID NO: 6.

In a sixth object, the present invention discloses the use of the hybrid protein, as defined above, or the hybrid nucleotide sequence, as previously defined, for the preparation of a medicament for the treatment of lesions and/or cancer caused by human papillomavirus (HPV) infection.

In one embodiment, the hybrid protein or nucleotide sequence, as previously defined, is used in the preparation of a medicament for the treatment of lesions and/or cancer caused by HPV-16 infection.

In one embodiment, the medicament is in the form of a therapeutic vaccine.

In a seventh object, the present invention presents a formulation comprising at least one hybrid nucleotide sequence selected from the group consisting of SEQ ID NO: 5, SEQ ID NO: 6, SEQ ID NO: 17 and/or SEQ ID NO: 18, and at least one pharmaceutically acceptable excipient.

In an eighth object, the present invention presents a formulation comprising at least one hybrid protein selected from the group consisting of SEQ ID NO: 2 and/or SEQ ID NO: 3, and at least one pharmaceutically acceptable excipient.

In one embodiment, the concentration of hybrid proteins in the formulations is from 1 µg to 70 µg. Preferably, from 20 µg to 70 µg. More preferably, from 40 µg to 70 µg. Even more preferably, 60 µg.

In one embodiment, the formulation is prepared for administration by nasal, intramuscular, subcutaneous, or intradermal route. Preferably, by subcutaneous route.

In a ninth object, the present invention presents a process for producing a formulation, comprising a step of mixing the hybrid protein or nucleotide sequence, as defined above, with at least one pharmaceutically acceptable excipient.

In a tenth object, the present invention provides a method for treating lesions and/or cancer caused by human papillomavirus (HPV) infection comprising administering a hybrid protein or a nucleotide sequence, as defined above.

In an eleventh object, the present invention presents a hybrid protein or a hybrid sequence, as defined above, for use as a medicament.

In a twelfth object, the present invention presents a hybrid protein or a hybrid sequence, as defined above, for use in the treatment of lesions and/or cancer caused by human papillomavirus (HPV) infection.

Degenerate sequences of the nucleotide sequences described above, which encode the same product, are also objects of the invention or, at least, considered equivalent means to those disclosed in this patent application.

Definitions of:
Nucleotide sequence: refers to the sequence of basic units (nucleotides) that form molecules of deoxyribonucleic acid (DNA) and ribonucleic acid (RNA). In the case of DNA, the nucleotide is composed of the phosphate group, a deoxyribose, and a nitrogenous base selected from adenine (A), thymine (T), cytosine (C), or guanine (G). In RNA, the nucleotide is composed of a phosphate group, a ribose, and a nitrogenous base selected from adenine (A), uracil (T), cytosine (C), or guanine (G). Furthermore, these sequences are degenerate, i.e., more than one type of codon can define a single amino acid, so different sequences can produce the same protein. In this context, a serine can be specified by AGY (which includes AGC and AGT) or TCN (which includes TCA, TCC, TCG, and TCT); a leucine can be specified by TTR (which includes TTA and TTG) or CTN (which includes CTA, CTC, CTG, and CTT); an arginine can be determined by CGN (which includes CGA, CGC, CGG, and CGT) or AGR (which includes AGA and AGG).

Formulation: refers to the combination of at least one active ingredient with at least one excipient. Formulations may be in solid form, such as a powder for reconstitution, or in liquid form, such as solutions and emulsions. Preferably, the formulation is a sterile vaccine that is available for administration by nasal, intramuscular, intradermal, or subcutaneous routes.

Excipient: refers to any compound of a formulation that is not the active ingredient or compound. These excipients assist in the proper delivery of the active ingredient and in enhancing its effect to achieve the desired purpose. Non-limiting examples that may be used in the context of this patent application include: vehicles, such as PBS; adjuvants, such as aluminum salts, saponins, and squalene-based oil-in-water emulsions (e.g., mf59^{®}), among others.

The extender sequences inserted in the N-terminal region of the constructs in this application contribute to the stability of the molecules and the exposure of the binding region to the HVEM receptor (see SEQs IDs No: 8 and 13). This binding to the HVEM receptor, via gD, triggers the activation of various cell types in the immune system. Thus, the hybrid proteins of the present patent application defined by SEQ ID NO: 2 and SEQ ID NO: 3 and their corresponding nucleotide sequences, both DNA and messenger RNA, can be used in the preparation of a medicament, preferably in the form of a vaccine with enhanced therapeutic and immunogenic potential, for the treatment of lesions and/or cancer caused by HPV infection.

### Examples

The examples shown here are intended only to illustrate one of the numerous ways of carrying out the invention, without, however, limiting its scope.

### Example 1 - Cloning of recombinant proteins

The nucleotide sequences of the recombinant proteins were used for cloning into an expression vector in *Escherichia coli*: SEQ ID NO: 4 in the pET-30a vector and SEQ ID NO: 5 and SEQ ID NO: 6 in the pET-28a vector. Then, the *E. coli* strains BL21 (DE3) star (for expression of SEQ ID NO: 1) and BL21 (DE3) (for expression of SEQ ID NO: 2 and 3) were transformed with recombinant plasmids. A single colony was inoculated onto Luria Bertani (LB) medium containing the antibiotic Kanamycin, and the culture was incubated at 37 °C at 200 rpm. Strains were individually inoculated into 1 L of LB medium containing Kanamycin and cultured at 37 °C and 200 rpm. Protein expression was induced with 0.5 mM isopropyl beta-D-1-thiogalactopyranoside (IPTG) at 37 °C, 200 rpm for 4 h when the optical density at 600 nm (OD600) reached approximately 1.2. Bacterial sediments were collected by centrifugation and resuspended with lysis buffer followed by sonication. The precipitate after centrifugation was dissolved using a denaturing agent. The target proteins were obtained by two-step purification using inclusion body washing and a Superdex 200 column (SEQ ID No: 1) or a His Trap HP 5ml affinity column (SEQ ID No: 2 and SEQ ID No: 3). Then, the target proteins were sterilized by a 0.22 µm filter before being stored in aliquots. The final concentration of the purified products was determined by SDS-PAGE quantification using a standard protein with a known concentration (Table 1).

**Table 1 - Final yield of hybrid protein production in prokaryotic cells (E. coli), after upstream and downstream processes from shaker culture on a laboratory scale. Quantification was performed in SDS-PAGE using a standard protein with known concentration.**

| **Protein** | **Yield** |
|---|---|
| SEQ ID NO: 1 | 8 mg/L of culture |
| SEQ ID NO: 2 | 4 mg/L of culture |
| SEQ ID NO: 3 | 60 mg/L of culture |

### Example 2 - Determination of purity, molecular weight and characterization of recombinant proteins.

To determine the purity, molecular weight, and characterization of recombinant proteins, SDS-PAGE (Fig. 2A) and western blot (Fig. 2A and 2C) assays were performed. The results confirmed an approximate molecular weight of 50kDa for the three proteins, with the one with SEQ ID No: 3 demonstrating the highest degree of purity and absence of "contaminating" bands. Western blot results also confirm the presence of histidine in the formulations (Fig. 2B), where it is possible to observe the presence of a more intense band for the protein with SEQ ID No: 2, which, unlike the others, has two tails of this amino acid. The recombinant protein from SEQ ID No: 1 showed lower recognition of the histidine tail, consistent with challenges encountered during purification by nickel affinity chromatography. All proteins showed recognition of gD (Fig. 2C), revealed with an anti-gD monoclonal antibody (1D-3; recognizes the interaction region with the HVEM cellular receptor) by chemiluminescence technique.

### Example 3 - In silico prediction of secondary structure

Amino acid sequences were used to perform in silico prediction of secondary structure using the AlphaFold artificial intelligence program and its database, designed as a machine learning system. The results for the structures related to sequences SEQ ID No: 1, SEQ ID No: 2, and SEQ ID No: 3 are presented in Fig. 1A, 1B, and 1C, respectively. The images were edited so that the portion corresponding to glycoprotein D was dark gray, the portion of HPV-16 oncoprotein E7 was medium gray, and the extender amino acids were light gray. In the predictions of the hybrid proteins SEQ ID No: 2 and SEQ ID No: 3, it is possible to observe better stabilization of the structure, with the formation of alpha helices of beta-pleated sheets. Furthermore, the conformation in these two hybrid proteins favors greater exposure of the E7 protein (in medium gray), which may result in better epitope presentation and, consequently, an increased immune response.

### Example 4 - Validation of the immunomodulatory role

To validate the immunomodulatory role of the proteins, they were evaluated for their ability to activate human monocyte-differentiated dendritic cells (MoDCs). For this step, blood samples from three healthy donors were used, collected by apheresis, from which monocytes were separated in a Ficoll gradient, enriched by adherence at 37 °C for 2 hours and cultured for 5 days with the cytokines GM-CSF and IL-4 in AIM V medium (ThermoFisher). On day 5, stimuli were added to the MoDCs. After 48 hours of exposure, the MoDCs were analyzed for their membrane phenotype and functional status. To determine the membrane phenotype, the cells were labeled with human antibodies conjugated to specific fluorophores (CD11c-PerCPCy5.5, HLA-DR-APCCy7, CD80-BV605 and CD83-PE) and evaluated by flow cytometry on the LSR Fortessa instrument (BD Biosciences) using FlowJo software (Tree Star) to analyze the percentage of CD11c+HLA-DR+ cells expressing the co-stimulatory molecules (Fig. 3A and 3B). Functional status was assessed by quantifying cytokines secreted in the culture supernatant 48h after stimulation (Fig. 3C and 3D) using the CBA Human Th1/Th2/Th17 kit (BD Biosciences, catalog 560485).

LPS-activated MoDCs were considered as a positive control and non-activated MoDCs (iDCs) as a negative control. MoDCs were considered activated with the recombinant protein when the expression of CD80 and CD83 was increased relative to iDCs. The results show that only the recombinant proteins SEQ ID No: 2 and SEQ ID No: 3 were able to induce the upregulation of CD80 and CD83 molecules in all individuals, with concomitant secretion of the pro-inflammatory cytokines TNF-alpha and IL-6.

Recombinant proteins were also evaluated for their antitumor effect against HPV-16-induced tumors in mice. In the subcutaneous tumor model, 1x10⁵ TC-1 cells were injected into the right flank of the animals, followed by the administration of one of the recombinant proteins, also subcutaneously, into the left flank, one day later. A second dose of protein was administered at a 7-day interval. Tumor growth results were obtained by measuring the minor and major diameters to calculate the tumor area (Fig. 4A-4E) of untreated (control) animals and animals treated with 60µg per dose of recombinant protein. Additionally, a group of animals treated with equimolar amounts of co-administered gD (SEQ ID NO: 21) and E7 (SEQ ID NO: 22) proteins was included, with the aim of evaluating the impact of the gene fusion strategy on recombinant proteins.

The data show that, overall, treating the animals with recombinant proteins led to a benefit in controlling tumor growth (Fig. 4A-4E). The results also demonstrate that the antitumor effect is only obtained when there is genetic fusion of gD with the target protein, in this case E7 of HPV-16, since treatment involving the co-administration of purified proteins obtained in isolation was unable to control tumor growth in any of the treated animals, as well as in the untreated control group. Regarding the different proteins, it was observed that those with SEQ ID NO: 1 and SEQ ID NO: 2 induced partial antitumor control, with 8 out of 11 animals and 6 out of 10 treated animals showing protection, respectively. On the other hand, the SEQ ID NO: 3 protein was able to lead to total tumor control in all treated animals, generating the best therapeutic protection results compared to the other proteins.

For survival analysis, a maximum tumor diameter of 15 mm was determined at which point the animals were euthanized. Thus, the data reveal that the groups treated with the SEQ ID NO: 1 protein or with the SEQ ID NO: 2 protein showed an increase in survival compared to untreated controls or those treated with co-administration of gD and E7, reaching 70% (Fig. 5A). In contrast, the group treated with the SEQ ID NO: 3 protein showed 100% survival and 100% tumor-free animals (Fig. 5A and 5B) at the end of the 60-day monitoring period.

Furthermore, to complement the results of tumor growth control, an evaluation of the specific cellular response was performed by analyzing the frequency of specific systemic CD8⁺ T lymphocytes, which are characterized by the intracellular production of IFN-gamma and TNF-alpha. The analysis was performed using individual blood samples collected seven days after the second dose, and the percentage of CD3⁺CD8⁺IFN-γ and CD3⁺CD8⁺TNF-α T cells from each animal was calculated decreasing the frequency of stimulated cells compared to the frequency of unstimulated cells with the E7-specific peptide (SEQ ID NO: 7) (Fig. 6A and 6B).

The results reveal an increased systemic response of CD8⁺ T lymphocytes that produce IFN-gamma E7-specific primarily present in the groups treated with SEQ ID NO: 1 and SEQ ID NO: 3 proteins, where the one with SEQ ID NO: 3 showed greater homogeneity in the frequency data of CD3⁺CD8⁺IFN-γ⁺ and CD3⁺CD8⁺TNFα⁺ cells in relation to the one with SEQ ID No: 1. Furthermore, the cellular response results corroborate the data obtained from antitumor control for the group treated with co-administered (non-fused) gD and E7 proteins, which did not show an increase in circulating effector cells under the tested conditions.

Therefore, overall, these results demonstrate the surprising immunogenic and therapeutic potential of the SEQ ID NO: 2 and SEQ ID NO: 3 proteins and the benefit of these optimizations is mainly related to controlling tumor growth in mice and the ability to activate human MoDCs.

Those skilled in the art will appreciate the knowledge presented here and will be able to reproduce the invention in the embodiments presented and in other variants and alternatives, covered by the scope of the following claims.

## Claims

1. Hybrid protein **characterized in that** it comprises an extender portion linked to the N- and/or C-terminal region from a modified portion of HSV-1 glycoprotein D fused to HPV oncoprotein E7.

2. Hybrid protein according to claim 1 **characterized in that** it comprises a sequence AA₁-AA₂-(AA₃)ₙ-(AA₄)ₙ-AA₅-(AA₃)ₙ-(AA₄)ₙ-(AA₆)ₘ-(AA₇)ₚ-AA₈-(AA₉)_{q}-(AA₁₀)ᵣ-(AA₁₁)ₛ, wherein:
AA₁ is an extender portion containing between 10 and 35 amino acids;
AA₂ is a portion of HSV-1 glycoprotein D between amino acids 26 to 269 or 21 to 269;
AA₃ is a nonpolar amino acid;
AA₄ is a polar amino acid;
AA₅ is the HPV-16 E7 antigen between amino acids 1 and 98, in which, optionally, the residues at positions 24 and 26 are replaced by a nonpolar amino acid;
AA₆ is a nonpolar amino acid;
AA₇ is a polar amino acid;
AA₈ is a portion of HSV-1 glycoprotein D between amino acids 270 to 341 or 268 to 339;
AA₉ is a nonpolar amino acid;
AA₁₀ is a polar amino acid;
AA₁₁ is a tail containing 6 histidines;
n, m, p, q, r, s are, independently, an integer from 0 to 1.

3. Hybrid protein according to claim 1 or 2 **characterized in that** the extender portion is defined by SEQ ID NO: 8 or SEQ ID NO: 13.

4. Hybrid protein according to one of claims 1 to 3 **characterized in that** it is defined by SEQ ID NO: 2 or SEQ ID NO: 3.

5. Hybrid nucleotide sequence **characterized in that** it comprises a coding sequence of an extender moiety linked to a modified HSV-1 glycoprotein D coding sequence fused to an HPV E7 protein coding sequence.

6. Hybrid nucleotide sequence according to claim 5 **characterized in that** it is defined by SEQ ID NO: 19, wherein:
R is A or G;
Y is C or T;
H is A, C or T;
N is A, C, G or T;
T is thymine; and
Optionally, one or more AGY codons are replaced by TCN, one or more TTR are replaced by CTN, and one or more CGN are replaced by AGR.

7. Hybrid nucleotide sequence according to claim 6 **characterized in that** it is a DNA nucleotide sequence defined by SEQ ID NO: 5.

8. Hybrid nucleotide sequence according to claim 5 **characterized in that** it is defined by SEQ ID NO: 23, wherein:
R is A or G;
Y is C or T;
H is A, C or T;
N is A, C, G or T;
T is uracil; and
Optionally, one or more AGY codons are replaced by TCN, one or more TTR are replaced by CTN, and one or more CGN are replaced by AGR.

9. Hybrid nucleotide sequence according to claim 8 **characterized in that** it is an RNA nucleotide sequence defined by SEQ ID NO: 17.

10. Hybrid nucleotide sequence according to claim 5 **characterized in that** it is defined by SEQ ID NO: 20, wherein:
R is A or G;
Y is C or T;
H is A, C or T;
N is A, C, G or T;
T is thymine; and
Optionally, one or more AGY codons are replaced by TCN, one or more TTR are replaced by CTN, and one or more CGN are replaced by AGR.

11. Hybrid nucleotide sequence according to claim 10 **characterized in that** it is a DNA nucleotide sequence defined by SEQ ID NO: 6.

12. Hybrid nucleotide sequence according to claim 5 **characterized in that** it is defined by SEQ ID NO: 24, wherein:
R is A or G;
Y is C or T;
H is A, C or T;
N is A, C, G or T;
T is uracil; and
Optionally, one or more AGY codons are replaced by TCN, one or more TTR are replaced by CTN, and one or more CGN are replaced by AGR.

13. Hybrid nucleotide sequence according to claim 12 **characterized in that** it is an RNA nucleotide sequence defined by SEQ ID NO: 18.

14. Nucleic acid molecule **characterized by** the nucleotide sequence of SEQ ID NO: 5, or degenerate sequences thereof, that encode the amino acid sequence of SEQ ID NO: 2.

15. Nucleic acid molecule **characterized by** the nucleotide sequence of SEQ ID NO: 6, or degenerate sequences thereof, that encode the amino acid sequence of SEQ ID NO: 3.

16. Vector for the expression of a hybrid protein, as defined in one of claims 1 to 4, **characterized in that** it comprises a genetic selection marker and one or more promoters functionally linked to a recombinant nucleotide sequence selected from the group consisting of SEQ ID NO: 5 or SEQ ID NO: 6.

17. Use of the hybrid protein, as defined in one of claims 1 to 4, or of the hybrid nucleotide sequence, as defined in one of claims 5 to 15, **characterized in that** it is used to prepare a medicament for the treatment of lesions and/or cancer caused by human papillomavirus (HPV) infection.

18. Use of the hybrid protein or nucleotide sequence, according to claim 17, **characterized in that** HPV is HPV-16.

19. Use of the hybrid protein or nucleotide sequence, according to one of claims 17 and 18, **characterized in that** the medicament is in the form of a therapeutic vaccine.

20. Formulation **characterized in that** it comprises at least one hybrid nucleotide sequence selected from the group consisting of SEQ ID NO: 5, SEQ ID NO: 6, SEQ ID NO: 17 and/or SEQ ID NO: 18, and at least one pharmaceutically acceptable excipient.

21. Formulation **characterized in that** it comprises at least one hybrid protein selected from the group consisting of SEQ ID NO: 2 and/or SEQ ID NO: 3, and at least one pharmaceutically acceptable excipient.

22. Formulation according to claim 21 **characterized in that** the hybrid protein is at a concentration of 1 µg to 70 µg.

23. Formulation according to one of claims 20 to 22, **characterized in that** it is available in a form for administration by nasal, intramuscular, subcutaneous or intradermal route.

24. Process for producing a formulation **characterized in that** it comprises a step of mixing the hybrid protein, as defined in one of claims 1 to 4, or the nucleotide sequence, as defined in one of claims 5 to 15, with at least one pharmaceutically acceptable excipient.

25. Method for treating lesions and/or cancer caused by human papillomavirus (HPV) infection, **characterized in that** it comprises the administration of a hybrid protein, as defined in one of claims 1 to 4, or of a nucleotide sequence, as defined in one of claims 5 to 15.

26. Hybrid protein, as defined in one of claims 1 to 4, or a nucleotide sequence, as defined in one of claims 5 to 15, **characterized in that** it is for use as a medicament.

27. Hybrid protein, as defined in one of claims 1 to 4, or a nucleotide sequence, as defined in one of claims 5 to 15, **characterized in that** it is for use in the treatment of lesions and/or cancer caused by human papillomavirus (HPV) infection.
